# EUROPEAN PATENT SPECIFICATION

(11) **EP 3 263 096 B1**
(45) Date of publication and mention of the grant of the patent: **30.07.2025**
(21) Application number: 17177748.5
(22) Date of filing: 23.06.2017
(51) Int. Cl.: A61K 9/48, A61K 31/5377

(54) **PHARMACEUTICAL CAPSULE COMPOSITION OF RIVAROXABAN**
PHARMAZEUTISCHE KAPSELFORMULIERUNG VON RIVAROXABAN
COMPOSITION PHARMACEUTIQUE EN CAPSULE DE RIVAROXABAN

(30) Priority: 28.06.2016 TR 201609006
(43) Date of publication of application: 03.01.2018
(73) Proprietor: Sanovel Ilac Sanayi ve Ticaret A.S., Sisli - Istanbul (TR)
(72) Inventor: TÜRKYILMAZ, Ali, 34460 Istanbul (TR); ZENGINER, Sibel, 34460 Istanbul (TR); ÖZTÜRK, Erkin, 34460 Istanbul (TR)
(74) Representative: Genç Ilhan, Oznur

(56) References cited:
- EP-A1- 2 942 058
- WO-A1-2016/131896
- WO-A2-2010/146179

## Description

### Field of Invention

This invention relates to a novel pharmaceutical capsule composition comprising rivaroxaban with at least one pharmaceutically acceptable excipient. Moreover, this invention relates to an improved method of preparing the rivaroxaban capsule composition which is simple, fast, cost-effective, time saving and industrially feasible.

### Background of Invention

Anticoagulant drugs target various procoagulant factors in the coagulation pathway.

Presently there are numerous anticoagulant drugs which are widely available. One of them is direct factor Xa inhibitors which plays a central role in the cascade of blood coagulation by inhibiting factor Xa directly. In an exemplary embodiment, the direct factor Xa inhibitors is a member selected from rivaroxaban, apixaban, edoxaban, betrixaban, TAK-442, darexaban, and pro-drug s thereof.

Rivaroxaban (Formula I), which is already known from WO0147919, is an anticoagulant and the first orally active direct factor Xa inhibitor. Rivaroxaban is used to treat deep vein thrombosis (DVT) and pulmonary embolism (PE). It is also used to help prevent strokes or serious blood clots in people who have atrial fibrillation.

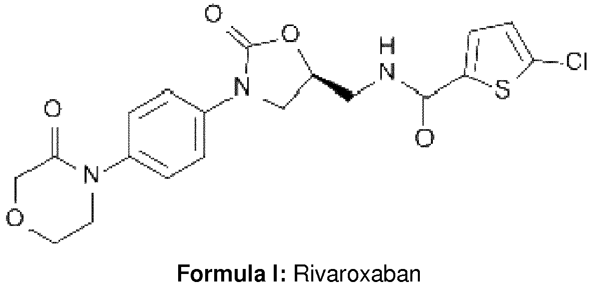

Rivaroxaban which is commercially available under the trade name Xarelto^{®}, is disclosed in US7157456 (B2), US7585860 (B2), US7592339 (B2). Also, U.S. Patent No. 7,157,456 provides rivaroxaban and processes for its preparation. WO 2010/146179 discloses a pharmaceutical composition comprising rivaroxaban in a capsule.

The prior art processes for the preparation of rivaroxaban compositions involve long reaction times and need use of expensive chemicals. Accordingly, these processes are not suitable on an industrial scale. Therefore, there is still a need in the art to develop economically attractive processes for the preparation of rivaroxaban compositions involving the use of less expensive chemicals and having fewer production steps. Thus, the present inventors have developed simple, fast, efficient and industrially feasible process for the preparation of rivaroxaban compositions.

### Detailed description of the invention

The main object of the present invention is to provide a new pharmaceutical capsule composition comprising with at least one pharmaceutically acceptable excipient which overcomes the above described problems in prior art and have additive advantages over them. The present invention is a pharmaceutical capsule formulation of rivaroxaban according to present claims comprising tricalcium citrate.

Another object of the present invention is to provide an improved method of preparing the rivaroxaban capsule composition which is simple, fast, cost-effective, time saving and industrially feasible.

Yet, another object of the present invention is to provide a method of preparing the rivaroxaban capsule composition which does not require to prepare wet or moist granulation. Thus, stability of the capsule composition is enhanced.

A further object of the invention is to use hydroxpropyl methylcellulose capsules instead of hard or soft gelatin capsules. In prior art the capsule formulations of rivaroxaban are hard or soft gelatin capsules and this cause crosslinking and agglomeration of the powder mixture and as a result of this, the decrease occurs in dissolution of the active ingredient rivaroxaban. This results, the decrease in bioavailability of the total capsule composition.

Another object of the invention is to provide a pharmaceutical composition for use in anticoagulant treatment and treating deep vein thrombosis (DVT) and pulmonary embolism (PE).

According to these objects, other features of the present invention are set forth herein more detailed.

According to this embodiment, the amount of rivaroxaban is between 1.0 to 60.0 % by weight of total formulation. Preferably the amount of rivaroxaban is between 5.0 to 50.0 % by weight of total formulation. More preferably the amount of rivaroxaban is between 5.0 to 40.0 % by weight of total formulation.

According to one embodiment, the active ingredient rivaroxaban has to be micronized and the particle size distribution of D (0.9) value is less than 20 µm. Preferably the particle size distribution of D (0.9) value of rivaroxaban is less than 10 µm This particle size distribution of D (0.9) value increased the dissolution of the active ingredient rivaroxaban and as a result the bioavailability of the capsule composition is enhanced.

As used here in, "particle size distribution" is defined by the cumulative volume size distribution as tested by a conventionally accepted method which is the laser diffraction method determined by the equipment of *Malvern Mastersizer 2000 laser diffraction particle size analyzer analyzer.* "D (0.9)" or "d₉₀" means that the size at which %90 by volume of the particles are finer. In this invention, particle size distribution of D (0.9) value of rivaroxaban has been measured with solid samples and D(0.9) value has been identified by Malvern Mastersizer 2000 laser diffraction particle size analyzer.

According to a further embodiment, at least one pharmaceutically acceptable excipient is fillers which are selected from the group comprising tricalcium citrate, lactose, microcrystalline cellulose, mannitol, spray-dried mannitol, dibasic calcium phosphate, dextrose, sucrose, dicalcium phosphate, sorbitol, xylitol, inositol, kaolin, inorganic salts, calcium salts, polysaccharides, sodium chloride, dextrates, lactitol, maltodextrin, sucrose-maltodextrin mixture, trehalose, sodium carbonate, sodium bicarbonate, calcium carbonate or mixtures thereof. Preferably fillers are tricalcium citrate, lactose, microcrystalline cellulose or mixtures thereof.

By the use of tricalcium citrate the fluidity of the powder mixture is increased in addition to its filler property. Moreover, tricalcium citrate further enhanced the binding properties of active ingredient rivaroxaban with povidone (polyvinyl pyrrolidone). It is also known from the prior art that, powder mixture of rivaroxaban is difficult to fill into capsules because of its poor solubility and poor binding characteristic because in the effective dose strength capsule size would result which are no longer swallowable. Thus in prior art most of the rivaroxaban formulations are tablet formulations instead of capsule formulations because of this disadvantage.

Besides this, while mixing the rivaroxaban with other chemicals or pharmaceutically acceptable excipients there is a need of moisture or wet granulation technics to overcome this problems but this may also cause stability problems and furthermore this makes the process less effective and longer. Because the active ingredient, rivaroxaban has a relatively poor water solubility. As a result of this, said difficulties occurs and this results with decreased bioavailability and bad stability results. Thus, the powder mixture is processed without need of wet or moist granulation and can be easily filled into capsules. As a result of this, the capsule composition has a good stability during its shelf-life.

In addition to these embodiments, to increase the bioavailability there are various methods in prior art but they are all problematic in various ways; such as using other solvents (i.e. ethanol, acetone) but rivaroxaban is also poor soluble in these solvents too, or using an alternative processes such as hydrophilization of active compound which results expensive use of chemicals and long process steps.

According to these embodiments, the amount of tricalcium citrate is between 0.10 to 30.0 % by weight of the total formulation. Preferably the amount of tricalcium citrate is between 0.50 to 20.0 % by weight of the total formulation. More preferably the amount of tricalcium citrate is between 1.0 to 10.0 % by weight of the total formulation.

In another embodiment the weight ratio of tricalcium citrate to active ingredient rivaroxaban is between 0.10 and 1.0 (w/w). Preferably the weight ratio is between 0.15 and 0.50 (w/w). More preferably the weight ratio is between 0.15 and 0.30 (w/w).

According to the challenges mentioned above the selection of the pharmaceutical acceptable excipients thus very important. According to this embodiment, one or more pharmaceutically acceptable excipient is selected from the group comprising binders, disintegrants, surface active agents lubricants, glidants or mixtures thereof.

Suitable binders may comprise but not limited to polyvinylpyrrolidone (povidone), polyethylene glycol, polyvinyl alcohol, starch, pregelatinized starch, glucose, glucose syrup, natural gums, sucrose, sodium alginate, hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

Suitable disintegrants may comprise but not limited to cross-linked carboxymethyl cellulose (croscarmellose sodium), cross-linked polyvinil pyrrolidone (crospovidone), low-substituted hydroxypropyl cellulose, pregelatinized starch, sodium carboxymethyl cellulose, calcium carboxymethyl cellulose, carboxymethyl cellulose, docusate sodium, polyacryline potassium, sodium alginate, corn starch, sodium starch glycolate, alginic acid, alginates, ion-exchange resins, magnesium aluminium silica, sodium dodesyl sulphate, sodium glycine carbonate or mixtures thereof.

Suitable surface active agents (surfactants) may comprise but not limited to sodium lauryl sulphate, sorbitan monolaurate, docusate sodium lauric acid, glyceril monooleate, myristyl alcohol, cetrimide macrogol 15 hydroxystearate or mixtures thereof.

Suitable lubricants may comprise but not limited to magnesium stearate, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate or mixtures thereof.

Suitable glidants may comprise but not limited to talc, colloidal silicon dioxide, aluminium silicate, colloidal silica, starch or mixtures thereof.

This invention is further defined by reference to the following example. Although the example is not intended to limit the scope of the present invention, it should be considered in the light of the description detailed above.

### Example: Rivaroxaban capsule

a. % 1.0 - 60.0 rivoraxaban
b. % 0.10 - 30.0 tricalcium citrate tetrahydrate
c. % 5.0 - 50.0 microcrystalline cellulose (pH 102)
d. % 5.0 - 50.0 lactose super tab 11 SD
e. % 0.10 - 30.0 povidone K25 (polyvinylpyrrolidone)
f. % 1.0 - 50.0 crosscarmellose sodium
g. % 0.10 - 1.0 sodium lauryl sulphate
h. % 0.50 - 5.0 colloidal silicon dioxide (aerosil 200)
i. % 0.10 - 2.0 magnesium stearate
by weight of total formulation.

### Production process:

Rivaroxaban, microcrystalline cellulose (pH 102), lactose super tab 11 SD, tricalcium citrate, povidone K25, crosscarmellose sodium and sodium lauryl sulphate are mixed 30 min.

The obtained powder mixture is sieved and colloidal silicon dioxide is added after it is also sieved and further mixed for 15 min. Then magnesium stearate is added to this powder mixture and further mixed for 5 min.

Finally the powder mixture is filled into hydroxypropyl methylcellulose capsules.

## Claims

1. A pharmaceutical capsule composition comprising rivaroxaban with at least one pharmaceutically acceptable excipient wherein the particle size distribution of rivaroxaban in diameter range with a D (0.9) value is less than 20 µm, measured with laser diffraction, and whereir the pharmaceutical capsule composition further comprises tricalcium citrate as a filler.

2. The pharmaceutical composition according to claim 1, wherein the amount of rivaroxaban is between 1.0 to 60.0 % by weight of total formulation.

3. The pharmaceutical composition according to claim 1 or 2, wherein the amount of tricalcium citrate is between 0.10 to 30.0 % by weight of the total formulation.

4. The pharmaceutical composition according to any of the preceding claims, wherein the weight ratio of tricalcium citrate to rivoraxaban is between 0.10 and 1.0 (w/w).

5. The pharmaceutical composition according to any preceding claims, further comprising binders, disintegrants, surface active agents, lubricants, glidants or mixtures thereof.

6. The pharmaceutical composition according to any preceding claims, comprising
a. % 1.0 - 60.0 rivoraxaban
b. % 0.10 - 30.0 tricalcium citrate
c. % 5.0 - 50.0 microcrystalline cellulose
d. % 5.0 - 50.0 lactose
e. % 0.10 - 30.0 polyvinyl pyrrolidone
f. % 1.0 - 50.0 crosscarmellose sodium
g. % 0.10 - 1.0 sodium lauryl sulphate
h. % 0.50 - 5.0 colloidal silicon dioxide
i. % 0.10 - 2.0 magnesium stearate
by weight of total formulation.

7. The pharmaceutical composition according to claim 6, wherein the powder mixture of said composition is filled into hydroxypropyl methylcellulose capsules.

## Patentansprüche

1. Pharmazeutische Kapselzusammensetzung, umfassend Rivaroxaban mit mindestens einem pharmazeutisch verträglichen Hilfsstoff, wobei die Teilchengrößenverteilung von Rivaroxaban im Durchmesserbereich mit einem D (0,9)-Wert weniger als 20 µm beträgt, gemessen mit Laserbeugung, und wobei die pharmazeutische Kapselzusammensetzung ferner Tricalciumcitrat als Füllstoff umfasst.

2. Pharmazeutische Zusammensetzung nach Anspruch 1, wobei die Menge an Rivaroxaban zwischen 1,0 und 60,0 Gew.-% der Gesamtformulierung beträgt.

3. Pharmazeutische Zusammensetzung nach Anspruch 1 oder 2, wobei die Menge an Tricalciumcitrat zwischen 0,10 und 30,0 Gew.-% der Gesamtformulierung beträgt.

4. Pharmazeutische Zusammensetzung nach einem der vorhergehenden Ansprüche, wobei das Gewichtsverhältnis von Tricalciumcitrat zu Rivoraxaban zwischen 0,10 und 1,0 (gew/gew) liegt.

5. Die pharmazeutische Zusammensetzung nach einem der vorhergehenden Ansprüche, die außerdem Bindemittel, Sprengmittel, oberflächenaktive Mittel, Schmiermittel, Gleitmittel oder Mischungen davon enthält.

6. Pharmazeutische Zusammensetzung nach einem der vorangehenden Ansprüche, umfassend
a. % 1,0 - 60,0 rivoraxaban
b. % 0,10 - 30,0 tricalciumcitrat
c. % 5,0 - 50,0 mikrokristalline Cellulose
d. % 5,0 - 50,0 laktose
e. % 0,10 - 30,0 polyvinylpyrrolidon
f. % 1,0 - 50,0 crosscarmellose-natrium
g. % 0,10 - 1,0 natriumlaurylsulfat
h. % 0,50 - 5,0 kolloidales siliziumdioxid
i. % 0,10 - 2,0 magnesiumstearat
nach Gewicht der gesamten Formulierung.

7. Pharmazeutische Zusammensetzung nach Anspruch 6, wobei die Pulvermischung der Zusammensetzung in Hydroxypropylmethylcellulosekapseln abgefüllt ist.

## Revendications

1. - Composition de capsule pharmaceutique comprenant du rivaroxaban avec au moins un excipient pharmaceutiquement acceptable, dans laquelle la distribution de la taille des particules de rivaroxaban dans la plage de diamètres avec une valeur D (0,9) est inférieure à 20 µm, mesurée par diffraction laser, et dans laquelle la composition de capsule pharmaceutique comprend en outre du citrate tricalcique en tant que charge.

2. - Composition pharmaceutique selon la revendication 1, dans laquelle la quantité de rivaroxaban est entre 1,0 et 60,0 % en poids de la formulation totale.

3. - Composition pharmaceutique selon l'une des revendications 1 ou 2, dans laquelle la quantité de citrate tricalcique est entre 0,10 et 30,0 % en poids de la formulation totale.

4. - Composition pharmaceutique selon l'une quelconque des revendications précédentes, dans laquelle le rapport pondéral du citrate tricalcique au rivoraxaban est entre 0,10 et 1,0 (p/p).

5. - Composition pharmaceutique selon l'une quelconque des revendications précédentes, comprenant en outre des liants, des désintégrants, des agents tensioactifs, des lubrifiants, des agents de glissement ou leurs mélanges.

6. - Composition pharmaceutique selon l'une quelconque des revendications précédentes, comprenant
a. 1,0 - 60,0 % de rivoraxaban
b. 0,10 - 30,0 % de citrate tricalcique
c. 5,0 - 50,0 % de cellulose microcristalline
d. 5,0 - 50,0 % de lactose
e. 0,10 - 30,0 % de polyvinyl pyrrolidone
f. 1,0 - 50,0 % de crosscarmellose sodique
g. 0,10 - 1,0 % de lauryl sulfate de sodium
h. 0,50 - 5,0 % de dioxyde de silicium colloïdal
i. 0,10 - 2,0 % de stéarate de magnésium
en poids de la formulation totale.

7. - Composition pharmaceutique selon la revendication 6, dans laquelle le mélange en poudre de ladite composition est introduit dans des capsules d'hydroxypropyl méthylcellulose.
